(19) Europäisches Patentamt
European Patent Office
Office européen des brevets

(11) **EP 3 439 473 B1**

(12) **EUROPÄISCHE PATENTSCHRIFT**

(45) Veröffentlichungstag und Bekanntmachung des Hinweises auf die Patenterteilung:
**07.08.2024 Patentblatt 2024/32**

(21) Anmeldenummer: **17716120.5**

(22) Anmeldetag: **30.03.2017**

(51) Internationale Patentklassifikation (IPC):
**A01N 43/80** (2006.01)   **A61K 8/49** (2006.01)
**C09D 7/63** (2018.01)   **C09D 5/14** (2006.01)
**A61Q 17/00** (2006.01)   **A61L 2/16** (2006.01)
**A01P 1/00** (2006.01)   **A01P 3/00** (2006.01)

(52) Gemeinsame Patentklassifikation (CPC):
(C-Sets verfügbar)
**A01N 43/80; A01P 1/00; A01P 3/00; A61K 8/27; A61K 8/34; A61K 8/345; A61K 8/347; A61K 8/37; A61K 8/42; A61K 8/44; A61K 8/49; A61K 8/4933; A61L 2/16; A61Q 17/005; C08K 5/0058;** (Forts.)

(86) Internationale Anmeldenummer:
**PCT/EP2017/000388**

(87) Internationale Veröffentlichungsnummer:
**WO 2017/174182 (12.10.2017 Gazette 2017/41)**

(54) **SYNERGISTISCHE BIOZIDZUSAMMENSETZUNGEN ENTHALTEND 5-CHLOR-2-METHYLISOTHIAZOLIN-3-ON**

SYNERGETIC BIOCIDAL COMPOSITIONS CONTAINING 5-CHLORO-2-METHYL-ISOTHIAZOLIN-3-ONE

COMPOSITIONS BIOCIDES SYNERGIQUES CONTENANT DE LA 5-CHLORO-2-MÉTHYLISOTHIAZOLIN-3-ONE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **05.04.2016 EP 16000774**

(43) Veröffentlichungstag der Anmeldung:
**13.02.2019 Patentblatt 2019/07**

(73) Patentinhaber: **THOR GmbH**
**67346 Speyer (DE)**

(72) Erfinder:
• **BAUM, Rüdiger**
**68809 Neulussheim (DE)**
• **WUNDER, Thomas**
**67435 Neustadt a.d. Weinstrasse (DE)**
• **SCHMIDT, Hans-Jürgen**
**67346 Speyer (DE)**

(56) Entgegenhaltungen:
EP-A1- 1 500 331   EP-A2- 2 025 228
WO-A1-2015/175241   WO-A1-2016/034266
WO-A2-2011/038316   CN-A- 102 268 840
CN-A- 104 306 178   CN-A- 104 306 178
CN-A- 105 400 421   JP-A- 2002 003 311
JP-A- H0 451 959   JP-A- H08 231 316
JP-A- H09 263 504   US-A- 3 929 562
US-A- 5 227 156   US-A- 5 227 156
US-A1- 2004 152 748   US-A1- 2004 152 748
US-A1- 2004 198 785   US-A1- 2004 198 785
US-A1- 2006 106 024   US-A1- 2006 106 024
US-A1- 2007 078 118   US-A1- 2009 163 445
US-A1- 2009 163 445   US-A1- 2014 121 253
US-A1- 2014 121 253

- HASEGAWA H ET AL: "Antibacterial agent for use in starch slurry, contains 2,2-dibromo-3-nitrilopropionamide, 2-bromo-2-nitropropane-1,3-diol and isothiazolone compound", WPI / 2017 CLARIVATE ANALYTICS,, vol. 2002, no. 39, 9 January 2002 (2002-01-09), XP002769891
- KAWAI K ET AL: "Antibacterial and anti-mould compsn. contg. nitro cpd. or propionamide cpd. - with tetrakis-phenol cpd. and iso-thiazolinone cpd. is useful as slime control agent and lesion inhibitor", WPI / 2017 CLARIVATE ANALYTICS,, vol. 1996, no. 46, 10 September 1996 (1996-09-10), XP002769893

(52) Gemeinsame Patentklassifikation (CPC): (Forts.)
**C08K 5/46; C09D 5/14; C09D 7/63;** A61K 2800/58

C-Sets
**A01N 43/80, A01N 43/80**

**Beschreibung**

Die vorliegende Erfindung betrifft eine Biozidzusammensetzung, die 5-Chlor-2-methylisothiazolin-3-on sowie 2-Methyl-1,2-Benzisothiazolin-3-on in einem Gewichtsverhältnis von 5-Chlor-2-methylisothiazolin-3-on zu 2-Methyl-1,2-Benzisothiazolin-3-on im Bereich von 1:5 bis 1:600 enthält, dadurch gekennzeichnet, dass die Zusammensetzung Methyl-4-isothiazolin-3-on im Bereich von 0 bis 2 Gew.-%, bezogen auf den Gesamtanteil des 5-Chlor-2-methyl-4-isothiazolin-3-ons, enthält. Die Erfindung betrifft ferner die Verwendung der erfindungsgemäßen Biozidzusammensetzungen zur Konservierung von technischen Produkten.

Technische, insbesondere wässrige Produkte, wie Farben, Lacke, Emulsionen und kosmetische Produkte werden üblicherweise unter Verwendung natürlicher, oder biologisch abbaubarer Rohstoffe hergestellt. Diese Rohstoffe und das als Lösemittel verwendete Wasser sind dabei häufig mit Keimen, wie Bakterien, Hefen und Pilzen, belastet. Verzichtet man bei der Herstellung dieser Produkte auf eine Konservierung, so können diese bereits einen Tag nach der Herstellung hohe Keimzahlen aufweisen.

Um sicherzustellen, dass diese Produkte den hygienischen Anforderungen gerecht werden und damit die Haltbarkeit der technischen Produkte gewährleistet ist, werden den Produkten so genannte Biozide hinzugegeben. Eines dieser Biozide ist das 5-Chlor-2-methylisothiazolin-3-on (CMIT). Dieses weist eine breite biozide Wirkung auf, hat aber bei der praktischen Handhabung verschiedene Nachteile. Beispielsweise kann diese Verbindung bei Personen, die damit umgehen, Allergien auslösen.

Um die Nachteile dieses zwar äußerst wirksamen, aber auch mit Nachteilen für den Endanwender behafteten CMITs zu überwinden, wurde in den 90er Jahren nach Alternativen Bioziden bzw. nach Biozidzusammensetzungen gesucht, deren Wirkstoffe synergistisch zusammenwirken und beim gleichzeitigen Einsatz in geringeren Konzentrationen verwendet werden können, verglichen mit den nötigen Konzentrationen im Falle der Einzelkomponente.

So kommt seit Anfang der 90er Jahre die in der EP 1 005 271 B1 offenbarte synergistische Mischung der Isothiazolinone 2-Methylisothiazolin-3-on (MIT) und 1,2-Benzisothiazolin-3-on (BIT) als Biozidzusammensetzung zum Einsatz. Diese Mischung wird von der Thor GmbH (Speyer, Bundesrepublik Deutschland) als Acticide MBS vertrieben, ist frei von 5-Chlor-2-methylisothiazolin-3-on und stellt zu heutiger Zeit den Standard bei der Gebindekonservierung von wasserbasierenden Farben und Putzen dar.

Ferner offenbart beispielsweise die europäische Offenlegungsschrift EP 0 676 140 als Ersatz für 5-Chlor-2-methylisothiazolin-3-on enthaltende Zusammensetzungen eine synergistische Biozidzusammensetzung, die 2-Methylisothiazolin-3-on und 2-n-Octylisothiazolin-3-on enthält.

Ebenfalls offenbart die EP-B 1 030 558 eine synergistische Biozidzusammensetzung, die 2-Methylisothiazolin-3-on und 3-Iod-2-propinyl-N-butylcarbamat enthält. Diese synergistische Zusammensetzung eignet sich bereits in geringen Konzentrationen zur Bekämpfung von Mikroorganismen.

Die internationale Offenlegungsschrift WO 2016/034266 A1 offenbart konservierte Produkte, die 1 bis 100 ppm 5-Chlor-2-methyl-4-isothiazolin-3-on mit einem Gehalt an 2-Methyl-4-isothiazolin-3-on im Bereich von 0 bis 2 Gew.-%, bezogen auf den Gesamtanteil des 5-Chlor-2-methyl-4-isothiazolin-3-ons enthalten.

Die internationale Offenlegungsschrift WO 2015/175241 A1 offenbart eine wässrige Zusammensetzung, die Isothiazolinone und tertiare Aminverbindungen enthält, sowie deren Verwendung in der Metallverarbeitung, in der Kosmetik, als Reinigungsmittel und als Holzbehandlungsmittel. Die Isothiazolinone, die in der Zusammensetzung zum Einsatz kommen, sind 2-Methyl-4-isothiazolin-3-on, 5-Chlor-2-methyl-4-isothiazolin-3-on, 2-Methyl-1,2-Benzisothiazolin-3-on und 2-Octyl-4-isothiazolin-3-on.

Die U.S. Offenlegungsschrift US 2014/121253 A1 offenbart synergistische antibakterielle Zusammensetzungen, die 2-Methyl-4-isothiazolin-3-on, 5-Chlor-2-methyl-4-isothiazolin-3-on und 2-Methyl-1,2-Benzisothiazolin-3-on enthalten. Synergistische antibakterielle Zusammensetzungen, die 2-Methyl-4-isothiazolin-3-on und 2-Methyl-1,2-Benzisothiazolin-3-on enthalten, werden in der U.S. Offenlegungsschrift US 2004/198785 A1 offenbart. Die in diesem Dokument beschriebenen Zusammensetzungen enthalten kein 5-Chlor-2-methyl-4-isothiazolin-3-on.

Ausgehend von dem vorstehend diskutierten Stand der Technik, besteht Bedarf an weiteren Biozidzusammensetzungen, deren Komponenten synergistisch zusammenwirken und deshalb beim gleichzeitigen Einsatz in geringeren Konzentrationen verwendet werden können, verglichen mit den nötigen Konzentrationen im Falle der Einzelkomponenten.

Diese Aufgabe löst die Erfindung durch eine Biozidzusammensetzung, enthaltend 5-Chlor-2-methylisothiazolin-3-on sowie 2-Methyl-1,2-Benzisothiazolin-3-on in einem Gewichtsverhältnis von 5-Chlor-2-methylisothiazolin-3-on zu 2-Methyl-1,2-Benzisothiazolin-3-on im Bereich von 1:5 bis 1:600. Die erfindungsgemäße Biozidzusammensetzung ist dadurch gekennzeichnet, dass sie Methyl-4-isothiazolin-3-on im Bereich von 0 bis 2 Gew.-%, bezogen auf den Gesamtanteil des 5-Chlor-2-methyl-4-isothiazolin-3-ons, enthält.

Die erfindungsgemäße Biozidzusammensetzung hat den Vorteil, dass das 5-Chlor-2-methylisothiazolin-3-on und das 2-Methyl-1,2-Benzisothiazolin-3-on synergistisch zusammenwirken, und deshalb beim gleichzeitigen Einsatz in geringen Konzentrationen verwendet werden können, verglichen mit niedrigen Konzentrationen im Falle der Einzelkomponenten.

Die vorliegende Erfindung ermöglicht es ferner, unter Verwendung des äußerst wirksamen 5-Chlor-2-methylisothiazolin-3-ons, das frei bzw. naherzu frei von 2-Methylisothiazolin-3-on ist, Produkte mit zu konservieren, und diese vor dem Inverkehrbringen so zu behandeln, dass das 5-Chlor-2-methylisothiazolin-3-on in dem Endprodukt nicht mehr vorhanden ist, so dass das von diesen Produkten ausgehende Allergierisiko für den Endanwender deutlich reduziert ist.

Die erfindungsgemäße Biozidzusammensetzung ist allgemein dadurch gekennzeichnet, dass sie Methyl-4-isothiazolin-3-on im Bereich von 0 bis 2 Gew.-%, bezogen auf den Gesamtanteil des 5-Chlor-2-methyl-4-isothiazolin-3-ons, enthält. Gemäß einer bevorzugten Ausführungsform der Erfindung ist die Biozidzusammensetzung dadurch gekennzeichnet, dass sie Methyl-4-isothiazolin-3-on im Bereich von 0 bis 1 Gew.-%, bezogen auf den Gesamtanteil des 5-Chlor-2-methyl-4-isothiazolin-3-ons, enthält. Gemäß einer besonders bevorzugten Ausführungsform ist die Biozidzusammensetzung dadurch gekennzeichnet, dass sie Methyl-4-isothiazolin-3-on im Bereich von 0 bis 0,5 Gew.-%, bezogen auf den Gesamtanteil des 5-Chlor-2-methyl-4-isothiazolin-3-ons, enthält.

"Wenigstens eine" bedeutet im Rahmen der vorliegenden Erfindung, dass die Biozidzusammensetzung eine einzige weitere Komponente bzw. Verbindung, oder mehrere, also zwei, drei, vier, fünf oder mehr Komponenten bzw. Verbindungen aus der jeweiligen Gruppe enthält.

Die Erfindung betrifft eine Biozidzusammensetzung, enthaltend 5-Chlor-2-methylisothiazolin-3-on sowie 2-Methyl-1,2-Benzisothiazolin-3-on in einem Gewichtsverhältnis von 5-Chlor-2-methylisothiazolin-3-on zu 2-Methyl-1,2-Benzisothiazolin-3-on im Bereich von 1:5 bis 1:600, bevorzugt im Bereich von 1:5 bis 1:100, besonders bevorzugt im Bereich von 1:5 bis 1:50. Die erfindungsgemäße Biozidzusammensetzung ist dadurch gekennzeichnet, dass sie Methyl-4-isothiazolin-3-on im Bereich von 0 bis 2 Gew.-%, bezogen auf den Gesamtanteil des 5-Chlor-2-methyl-4-isothiazolin-3-ons, enthält.

Eine Biozidzusammensetzung, enthaltend ausschließlich 5-Chlor-2-methylisothiazolin-3-on mit einem Gehalt an Methyl-4-isothiazolin-3-on im Bereich von 0 bis 2 Gew.-%, bezogen auf den Gesamtanteil des 5-Chlor-2-methyl-4-isothiazolin-3-ons, und 2-Methyl-1,2-Benzisothiazolin-3-on, in den jeweils angegebenen Gewichtsverhältnissen ohne Vorliegen einer weiteren Komponente, wird im Rahmen der vorliegenden Erfindung als "erfindungsgemäße Biozidzusammensetzung" bezeichnet werden.

Eine "erfindungsgemäße Biozidzusammensetzung" kann neben dem 5-Chlor-2-methylisothiazolin-3-on mit einem Gehalt an Methyl-4-isothiazolin-3-on im Bereich von 0 bis 2 Gew.-%, bezogen auf den Gesamtanteil des 5-Chlor-2-methyl-4-isothiazolin-3-ons, und dem 2-Methyl-1,2-Benzisothiazolin-3-on zusätzlich ein oder mehrere weitere Bestandteile aufweisen. Der oder die weiteren Bestandteile können dabei eine mikrobizide Wirkung aufweisen, oder sie können keine mikrobizide Wirkung aufweisen, also etwa ein Lösungsmittel, Dispergiermittel oder Suspensionsmittel sein.

Es ist vorteilhaft, wenn die erfindungsgemäße Biozidzusammensetzung das 5-Chlor-2-methylisothiazolin-3-on mit einem Gehalt an Methyl-4-isothiazolin-3-on im Bereich von 0 bis 2 Gew.-%, bezogen auf den Gesamtanteil des 5-Chlor-2-methyl-4-isothiazolin-3-ons, und das 2-Methyl-1,2-Benzisothiazolin-3-on in einer Gesamtkonzentration von 0,5 bis 50 Gew.-%, bevorzugt von 1 bis 20 Gew.-%, besonders bevorzugt von 2,5 bis 10 Gew.-%, jeweils bezogen auf die gesamte Biozidzusammensetzung, enthält.

In einer weiteren Ausführungsform besteht die erfindungsgemäße Biozidzusammensetzung überwiegend aus 5-Chlor-2-methylisothiazolin-3-on mit einem Gehalt an Methyl-4-isothiazolin-3-on im Bereich von 0 bis 2 Gew.-%, bezogen auf den Gesamtanteil des 5-Chlor-2-methyl-4-isothiazolin-3-ons, und dem 2-Methyl-1,2-Benzisothiazolin-3-on (also der erfindungsgemäßen Biozidzusammensetzung bzw. dem Biozidgemisch). Dies bedeutet, dass die erfindungsgemäße Biozidzusammensetzung als Hauptbestandteil das erfindungsgemäße Biozidgemisch, vorzugsweise in einer Menge von gleich oder größer 50 Gew.-%, mehr bevorzugt in einer Menge von gleich oder größer 70 Gew.-%, insbesondere in einer Menge von gleich oder größer 90 Gew.-%, insbesondere in einer Menge von gleich oder größer 95 Gew.-%, bezogen auf die Gesamtmasse an biozidem Wirkstoff, enthält. Daneben kann mindestens ein weiteres Biozid vorhanden sein, ebenso wie ein oder mehrere Lösungsmittel, Dispergiermittel oder Suspensionsmittel.

Gemäß einer weiteren Ausführungsform der Erfindung besteht die erfindungsgemäße Biozidzusammensetzung im Wesentlichen aus dem erfindungsgemäßen Biozidgemisch, d.h., dass neben dem Gemisch wohl noch ein oder auch mehrere andere Biozide enthalten sein können, diese aber in einer solchen Menge vorliegen, in der kein Beitrag des jeweiligen von den Komponenten des Gemischs verschiedenen Biozids zum Gesamteffekt der entstehenden Mischung vorliegt. Wenn also die biozide Wirkung einer erfindungsgemäßen Biozidzusammensetzung, die - neben den Komponenten des erfindungsgemäßen Biozidgemischs als wesentlichem Bestandteil - noch ein oder mehrere weitere Biozide in untergeordneter bzw. geringfügigerer Konzentration aufweist, nicht verändert ist gegenüber dem Einsatz des erfindungsgemäßen Biozidgemischs, wird dieses im Zusammenhang mit der vorliegenden Erfindung als "im Wesentlichen bestehend" bezeichnet. Es können ein oder mehrere weitere Bestandteile ohne eine biozide Wirkung vorhanden sein, etwa Lösungsmittel.

In einer weiteren Ausführungsform kann die erfindungsgemäße Biozidzusammensetzung aus den Komponenten des erfindungsgemäßen Biozidgemischs als einzigen bioziden Wirkstoffen bestehen, also einem Wirkstoffgehalt von 100 % 5-Chlor-2-methylisothiazolin-3-on mit einem Gehalt an Methyl-4-isothiazolin-3-on im Bereich von 0 bis 2 Gew.-%, bezogen auf den Gesamtanteil des 5-Chlor-2-methyl-4-isothiazolin-3-ons, und 2-Methyl-1,2-Benzisothiazolin-3-on. In einem solchen Fall ist es lediglich möglich, dass ein oder mehrere weitere Bestandteile ohne eine biozide Wirkung vorhanden sind, etwa Lösungsmittel bzw. Stabilisierungsmittel.

Gemäß einer Ausführungsform der Erfindung liegt die erfindungsgemäße Biozidzusammensetzung als Konzentrat vor, welches den damit zu konservierenden Stoffen bzw. Produkten zugegeben wird. Das Konzentrat weist vorteilhafterweise das 5-Chlor-2-methylisothiazolin-3-on in einer Menge von 0,5 bis 50 Gew.-%, bevorzugt in einer Menge von 0,5 bis 20 Gew.-%, besonders bevorzugt in einer Menge von 0,7 bis 10 Gew.-%, bezogen auf die Gesamtmasse des Konzentrats, auf. Die Menge an 2-Methyl-1,2-Benzisothiazolin-3-on, die in dem Konzentrat enthalten ist, beträgt 0,5 bis 50 Gew.-%, bevorzugt 1 bis 20 Gew.-%, besonders bevorzugt 1 bis 5 Gew.-%, bezogen auf die Gesamtmasse des Konzentrats.

In einer vorteilhaften Ausführungsform liegt die Biozidzusammensetzung in Form einer flüssigen Zubereitung vor, beispielsweise als Lösung, Suspension oder Dispersion in einem flüssigen Medium. Selbstverständlich kann die erfindungsgemäße Biozidzusammensetzung auch direkt in einem zu konservierenden Produkt gemischt werden. Dies geschieht durch Zugabe der einzelnen mikrobiziden Wirkkomponenten zu dem zu konservierenden Produkt.

Es ist zweckmäßig, wenn die erfindungsgemäße Biozidzusammensetzung für die Anwendung in Kombination mit einem polaren oder unpolaren flüssigen Medium vorliegt.

Bevorzugte polare flüssige Medien sind Wasser, aliphatische Alkohole mit 1 bis 4 Kohlenstoffatomen, zum Beispiel Ethanol und Isopropanol, ein Glykol, zum Beispiel Ethylenglykol, Diethylenglykol, 1,2-Propylenglykol, Dipropylenglykol und Tripropylenglykol, ein Glykolether, wie Butylglykol und Butyldiglykol, ein Glykolester, wie Butylglykolacetat oder 2,2,4-Trimethylpentandiolmonoisobutyrat, ein Polyethylenglykol, ein Propylenglykol, N,N-Dimethylformamid, oder ein Gemisch aus zwei oder mehr solcher Medien. Das polare flüssige Medium ist insbesondere Wasser und/oder Glykol.

Als unpolare flüssige Medien können beispielsweise Aromate, vorzugsweise Xylol und Toluol dienen, auch diese können alleine oder als Gemische aus zwei oder mehreren solcher Medien eingesetzt werden.

Die erfindungsgemäße Biozidzusammensetzung kann auch gleichzeitig mit einem polaren oder einem unpolaren flüssigen Medium kombiniert werden.

Allgemein ist es möglich, die erfindungsgemäße Biozidzusammensetzung durch den Zusatz weiterer Wirkstoffe speziellen Anwendungsgebieten anzupassen, beispielsweise im Sinne einer erhöhten Wirkung oder einer verbesserten Verträglichkeit mit den vor den Mikroorganismen schützenden Stoffen. Diese weiteren Stoffe sind dem Fachmann auf dem Gebiet der Biozide bekannt, und können von diesen in Abhängigkeit der Anwendung der erfindungsgemäßen Biozidzusammensetzung ausgewählt werden.

Die erfindungsgemäße Biozidzusammensetzung kann zur Konservierung auf sehr unterschiedlichen Gebieten eingesetzt werden. Bevorzugt wird die erfindungsgemäße Zubereitung für die Gebindekonservierung, bevorzugt für die Konservierung von Anstrichmitteln, wie Farben, Lacken und Putzen, von Polymerdispersionen, Emulsionen, Slurries, Pigmentpräparationen, Wasch- und Reinigungsmitteln, und Klebstoffen verwendet.

Die Erfindung betrifft weiterhin auch Produkte, die die erfindungsgemäße Biozidzusammensetzung enthalten. Diese Produkte sind allgemein ausgewählt aus Ligninsulfonaten und Stärkepräparation in Anstrichmitteln, Farben, Lacken, Lasuren und Putzen, Emulsionen, Latices, Polymerdispersionen, Kreideaufschlämmungen, mineralischen Slurrys, keramischen Massen, Klebstoffen, Duftstoffen, caseinhaltigen Produkten, stärkehaltigen Produkten, Bitumenemulsionen, Tensidlösungen, Kraftstoffen, Reinigungsmitteln, Pigmentpasten und Pigmentdispersionen, Tinten, lithographischen Flüssigkeiten, Verdickern, kosmetischen Produkten, Toilettenartikeln, Wasserkreisläufen, Flüssigkeiten bei der Holzverarbeitung, Flüssigkeiten bei der Erdölgewinnung, Flüssigkeiten bei der Papierverarbeitung, Flüssigkeiten bei der Lederherstellung, Flüssigkeiten bei der Textilherstellung, Bohr- und Schneidölen, hydraulischen Flüssigkeiten und Kühlschmierstoffen.

Bei der praktischen Anwendung kann die Biozidzusammensetzung entweder als fertiges Gemisch, oder durch getrennte Zugabe der einzelnen Komponenten der Zusammensetzung in das zu konservierende Produkt eingebracht werden.

Der Gehalt der Komponenten in der Konservierungsanwendung (die Anwendungskonzentration) kann über weite Bereiche hinweg je nach Anwendungszweck variieren und wird üblicherweise von dem Fachmann auf dem Gebiet der Biozide festgelegt. Die nachfolgend angegebenen Konzentrationsbereiche geben dem Fachmann einen Hinweis auf die einzusetzenden Konzentrationen.

In dem mit der erfindungsgemäßen Zusammensetzung konservierten Produkt ist die wenigstens eine Komponente 2-Methyl-1,2-Benzisothiazolin-3-on. Der Gehalt der Komponenten in der Konservierungsanwendung (die Anwendungskonzentration) kann über weite Bereiche hinweg je nach Anwendungszweck variieren und wird üblicherweise von dem Fachmann auf dem Gebiet der Biozide festgelegt. Die nachfolgend angegebenen Konzentrationsbereiche geben dem Fachmann einen Hinweis auf die einzusetzenden Konzentrationen.

In dem mit der erfindungsgemäßen Biozidzusammensetzung konservierten Produkt ist das Chlor-2-methylisothiazolin-3-on allgemein in einer Menge im Bereich von 1 bis 500 ppm, bevorzugt in einer Menge von 5 bis 200 ppm, bevorzugt in einer Menge von 10 bis 100 ppm, insbesondere bevorzugt in einer Menge von 10 bis 30 ppm, enthalten.

In dem mit der erfindungsgemäßen Zusammensetzung konservierten Produkt ist 2-Methyl-1,2-Benzisothiazolin-3-on enthalten.

Bei Anwesenheit von 2-Methyl-1,2-Benzisothiazolin-3-on ist dies in dem mit der erfindungsgemäßen Zusammensetzung konservierten Produkt allgmein in einer Menge im Bereich von 1 bis 1.000 ppm, bevorzugt in einer Menge von 5 bis 750 ppm, bevorzugt in einer Menge von 10 bis 500 ppm, insbesondere bevorzugt in einer Menge von 20 bis 250 ppm, enthalten.

**Die folgenden Beispiele dienen zur weiteren Veranschaulichung der vorliegenden Erfindung.**

Untersuchung der synergistischen Wirkung

Es wurde der Synergismus einer Kombination von 5-Chlor-2-methylisothiazolin-3-on, das 0,003 Gew.%, bezogen auf den Gesamtanteil des 5-Chlor-2-methyl-4-isothiazolin-3-ons, 2-Methylisothiazolin-3-on enthielt, mit 2-Methyl-1,2-Benzisothiazolin-3-on (M-BIT) auf eine synergistische Wechselwirkung hin untersucht.

Als Testorganismen wurden das Gram-negative Bakterium *Pseudomonas putida* (DSM 25068) verwendet. Dazu wurden Gemische mit unterschiedlichen Konzentrationen der jeweiligen Wirkstoffe hergestellt und bezüglich ihrer Wirkung auf Pseudomonas putida überprüft. Die Überprüfung erfolgte in Müller-Hinton-Bouillon MHB (pH 7.0) mit einer Zelldichte von $10^6$ Keimen pro ml.

Die Inkubationszeit der Mikrotiterplatten betrug 48 Stunden bei 30 °C. Nach 48 Stunden wurden die Ansätze auf Trübung durch Wachstum ausgewertet und zusätzlich die optische Dichte photometrisch bestimmt. Auf diese Weise wurden die minimalen Hemmkonzentrationen (MHK) der beiden Wirkstoffe einzeln und in Kombination bestimmt.

Der auftretende Synergismus wurde durch Berechnen des Synergieindex (SI) zahlenmäßig dargestellt. Die Berechnung erfolgte nach der gängigen Methode von F.C. Kull et al., Applied Microbiology, Bd. 9 (1961), S. 538. Dort wird der SI nach der folgenden Formel berechnet:

$$\text{Synergieindex SI} = Q_a/Q_A + Q_b/Q_B$$

$Q_a$ = Konzentration von Komponente A in der Mischung A + B
$Q_A$ = Konzentration von Komponente A als alleinigem Biozid
$Q_b$ = Konzentration von Komponente B in der Mischung A + B
$Q_a$ = Konzentration von Komponente B als alleinigem Biozid

Wenn der Synergieindex einen Wert von über 1 aufweist, bedeutet dies, dass ein Antagonismus vorliegt. Wenn der Synergieindex den Wert 1 annimmt, bedeutet dies, dass eine Addition der Wirkung der beiden Biozide /Verbindungen gegeben ist. Wenn der Synergieindex einen Wert von unter 1 annimmt, bedeutet dies, dass ein Synergismus der beiden Biozide besteht.

Wenn der Synergieindex einen Wert von über 1 aufweist, bedeutet dies, dass ein Antagonismus vorliegt.

Beispiel 1: Untersuchung der synergistischen Wechselwirkung zwischen Chlor-2-methyl-4-isothiazolin-3-on (CMIT) und 2-Methyl-1,2-Benzisothiazolin-3-on (M-BIT)

Berechnung des Synergieindexes von Chlor-2-methyl-4-isothiazolin-3-on (CMIT) und 2-Methyl-1,2-Benzisothiazolin-3-on (M-BIT) bezüglich *Pseudomonas putida* bei einer Inkubationszeit von 48 Stunden bei 30 °C.

Berechnung des Synergieindexes von Chlor-2-methyl-4-isothiazolin-3-on (CMIT) und 2-Methyl-1,2-Benzothiazol-3-on (M-BIT) bezüglich *Pseudomonas putida* bei einer Inkubationszeit von 48 Stunden bei 30 °C.

Tabelle 1

| Qa (CMIT) [ppm] | Qb (M-BIT) [ppm] | Synergie Index |
|---|---|---|
| 0 | 200 | 1,00 |
| 0,25 | 150 | 0,92 |
| 0,5 | 100 | 0,83 |
| 0,5 | 75 | 0,71 |
| 0,5 | 50 | 0,58 |
| 0,75 | 40 | 0,70 |

(fortgesetzt)

| Qa (CMIT) [ppm] | Qb (M-BIT) [ppm] | Synergie Index |
|---|---|---|
| 0,75 | 30 | 0,65 |
| 1 | 20 | 0,77 |
| 1 | 10 | 0,72 |
| 1 | 5 | 0,69 |
| 1,5 | 0 | 1,00 |
| Qa: Konzentration von CIT in der Mischung, die einen Endpunkt zeigt<br>QA: Konzentration von CIT als alleiniges Agens, das einen Endpunkt zeigt<br>Qb: Konzentration von M-BIT in der Mischung, die einen Endpunkt zeigt<br>QB: Konzentration von M-BIT als alleiniges Agens, das einen Endpunkt zeigt | | |

Aus der Tabelle 1 ist ersichtlich, dass der optimale Synergismus, d.h. der niedrigste Synergieindex (0,58) einer Biozidzusammensetzung aus CMIT und M-BIT bei einem Verhältnis von 0,5 ppm CMIT zu 50 ppm M-BIT liegt. Ein Synergismus lässt sich nachweisen, wenn das Gewichtsverhältnis der Biozide CMIT und M-BIT im Bereich von 1:5 bis 1:600 liegt.

**Patentansprüche**

1. Biozidzusammensetzung, enthaltend 5-Chlor-2-methylisothiazolin-3-on sowie 2-Methyl-1,2-Benzisothiazolin-3-on in einem Gewichtsverhältnis von 5-Chlor-2-methylisothiazolin-3-on zu 2-Methyl-1,2-Benzisothiazolin-3-on im Bereich von 1:5 bis 1:600,
**dadurch gekennzeichnet, dass** die Zusammensetzung Methyl-4-isothiazolin-3-on im Bereich von 0 bis 2 Gew.-%, bezogen auf den Gesamtanteil des 5-Chlor-2-methyl-4-isothiazolin-3-ons, enthält.

2. Biozidzusammensetzung nach Anspruch 1, **dadurch gekennzeichnet, dass** diese das 5-Chlor-2-methylisothiazolin-3-on sowie das wenigstens eine weitere Biozid in einer Gesamtkonzentration von 0,5 bis 50 Gew.-%, bezogen auf die gesamte Biozidzusammensetzung enthält.

3. Biozidzusammensetzung nach Anspruch 1 oder 2, **dadurch gekennzeichnet, dass** sie ein polares und/oder unpolares Medium umfasst.

4. Biozidzusammensetzung nach Anspruch 3, **dadurch gekennzeichnet, dass** sie als polares flüssiges Medium Wasser, einen aliphatischen Alkohol mit 1 bis 4 Kohlenstoffatomen, ein Glykol, einen Glykolether, einen Glykolester, ein Polyethylenglykol, ein Propylenglykol, N,N-Dimethylformamid, 2,2,4-Trimethylpentandiolmonoisobutyrat oder ein Gemisch aus solchen umfasst.

5. Biozidzusammensetzung nach einem der Ansprüche 1 bis 4, **dadurch gekennzeichnet, dass** sie flüssige Zubereitung vorliegt.

6. Verwendung der Biozidzusammensetzung nach einem der Ansprüche 1 bis 5 für die Gebindekonservierung.

7. Verwendung der Biozidzusammensetzung nach Anspruch 6 für die Gebindekonservierung von Anstrichmitteln wie Farben, Lacken und Putzen, von Polymerdispersionen, Emulsionen, Latices und Klebstoffen.

8. Konserviertes Produkt enthaltend eine Biozidzusammensetzung wie in einem der Ansprüche 1 bis 5 beschrieben, **dadurch gekennzeichnet, dass** das konservierte Produkt Methyl-4-isothiazolin-3-on im Bereich von 0 bis 2 Gew.-%, bezogen auf den Gesamtanteil des 5-Chlor-2-methyl-4-isothiazolin-3-ons, enthält.

9. Konserviertes Produkt nach Anspruch 8, ausgewählt aus Ligninsulfonaten und Stärkepräparation in Anstrichmitteln, Farben, Lacken, Lasuren und Putzen, Emulsionen, Latices, Polymerdispersionen, Kreideaufschlämmungen, mineralischen Slurrys, keramischen Massen, Klebstoffen, Duftstoffen, caseinhaltigen Produkten, stärkehaltigen Produkten, Bitumenemulsionen, Tensidlösungen, Kraftstoffen, Reinigungsmitteln, Pigmentpasten und Pigmentdispersionen, Tinten, lithographischen Flüssigkeiten, Verdickern, kosmetischen Produkten, Toilettenartikeln, Wasserkreisläufen, Flüssigkeiten bei der Holzverarbeitung, Flüssigkeiten bei der Erdölgewinnung, Flüssigkeiten bei der Papierverarbeitung, Flüssigkeiten bei der Lederherstellung, Flüssigkeiten bei der Textilherstellung, Bohr- und Schneidölen, hydraulischen Flüssigkeiten und Kühlschmierstoffen.

## Claims

1. A biocidal composition comprising 5-chloro-2-methylisothiazolin-3-one and 2-methyl-1,2-benzisothiazolin-3-one in a weight ratio of 5-chloro-2-methylisothiazolin-3-one to 2-methyl-1,2-benzisothiazolin-3-one in the range from 1:5 to 1:600,
   **characterized in that** the composition contains methyl-4-isothiazolin-3-one in the range from 0 to 2% by weight, in relation to the total proportion of 5-chloro-2-methyl-4-isothiazolin-3-one.

2. The biocidal composition according to claim 1, **characterized in that** it contains the 5-chloro-2-methylisothiazolin-3-one and the at least one further biocide in a total concentration of 0.5 to 50% by weight, based on the entire biocidal composition.

3. The biocidal composition according to claim 1 or 2, **characterized in that** it comprises a polar and/or non-polar medium.

4. The biocidal composition as claimed in claim 3, **characterized in that** it contains, as the polar liquid medium, water, an aliphatic alcohol with 1 to 4 carbon atoms, a glycol, a glycol ether, a glycol ester, a polyethylene glycol, a propylene glycol, N,N-dimethylformamide, 2,2,4-trimethylpentane diol monoisobutyrate or a mixture of these.

5. The biocidal composition as claimed in any one of claims 1 to 5, **characterized in that** it is present as a liquid preparation.

6. A use of the biocidal composition as claimed in any one of claims 1 to 5 for in can preservation.

7. The use of the biocidal composition as claimed in claim 6 for in can preservation of coating materials such as paints, varnishes and plasters, of polymer dispersions, emulsions, latexes and adhesives.

8. A preserved product containing the biocidal composition as claimed in any one of claims 1 to 5,
   **characterized in that** the preserved product contains methyl-4-isothiazolin-3-one in the range from 0 to 2% by weight, in relation to the total proportion of 5-chloro-2-methyl-4-isothiazolin-3-one.

9. The preserved product as claimed in claim 8, selected from lignin sulfonates and starch preparations in coating materials, paints, varnishes, glazes and plasters, emulsions, latexes, polymer dispersions, chalk slurries, mineral slurries, ceramics, adhesives, fragrances, casein-containing products, starch-containing products, bitumen emulsions, surfactant solutions, fuels, detergents, pigment pastes and pigment dispersions, inks, lithographic liquids, thickeners, cosmetic products, toilet articles, water systems, fluids in wood working, fluids in oil extraction, fluids in paper processing, fluids in leather production, fluids in textile production, drilling and cutting oils, hydraulic fluids and coolants.

## Revendications

1. Composition biocide contenant de la 5-chloro-2-méthylisothiazolin-3-one, ainsi que de la 2-méthyl-1,2-benzisothiazolin-3-one selon un rapport en poids de la 5-chloro-2-méthylisothiazolin-3-one à la 2-méthyl-1,2-benzisothiazolin-3-one dans la plage de 1:5 à 1:600, **caractérisée en ce que** la composition contient de la méthyl-4-isothiazolin-3-one dans la plage de 0 à 2 % en poids par rapport à la quantité totale de la 5-chloro-2-méthyl-4-isothiazolin-3-one.

2. Composition biocide selon la revendication 1, **caractérisée en ce qu'**elle contient la 5-chloro-2-méthylisothiazolin-3-one ainsi que l'au moins un autre biocide selon une concentration totale de 0,5 à 50 % en poids par rapport à la composition biocide totale.

3. Composition biocide selon la revendication 1 ou 2, **caractérisée en ce qu'**elle comprend un milieu polaire et/ou apolaire.

4. Composition biocide selon la revendication 3, **caractérisée en ce qu'**elle comprend comme milieu liquide polaire de l'eau, un alcool aliphatique ayant 1 à 4 atomes de carbone, un glycol, un éther de glycol, un ester de glycol, un polyéthylèneglycol, un propylèneglycol, du N,N-diméthylformamide, du monoisobutyrate de 2,2,4-triméthylpentanediol ou un mélange de ceux-ci.

5. Composition biocide selon l'une des revendications 1 à 4, **caractérisée en ce qu'**elle se présente sous forme d'une préparation liquide.

6. Utilisation de la composition biocide selon l'une des revendications 1 à 5, pour une conservation dans le bidon.

7. Utilisation de la composition biocide selon la revendication 6 pour la conservation dans le bidon de compositions de revêtements telles que des peintures, des vernis et des enduits, de dispersions polymères, d'émulsions, de latex et d'adhésifs.

8. Produit conservé contenant une composition biocide telle que décrite dans l'une des revendications 1 à 5, **caractérisé en ce que** le produit conservé contient de la méthyl-4-isothiazolin-3-one dans la plage de 0 à 2 % en poids par rapport à la quantité totale de la 5-chloro-2-méthyl-4-isothiazolin-3-one.

9. Produit conservé selon la revendication 8, choisi parmi les lignines sulfonate et les préparations d'amidon dans les compositions de revêtement, les peintures, les vernis, les lasures et les enduits, les émulsions, les latex, les dispersions polymères, les suspensions de craie, les minerais en boues, les masses céramiques, les adhésifs, les parfums, les produits contenant de la caséine, les produits contenant de l'amidon, les émulsions bitumineuses, les solutions de tensioactif, les carburants, les nettoyants, les pâtes pigmentaires et les dispersions pigmentaires, les encres, les liquides pour lithographie, les épaississants, les produits cosmétiques, les articles de toilette, les circuits d'eau, les liquides lors de la transformation du bois, les liquides lors de la récupération du pétrole, les liquides lors du traitement du papier, les liquides lors de la fabrication du cuir, les liquides lors de la fabrication de textiles, les fluides de forage et huiles de coupe, les liquides hydrauliques et les lubrifiants réfrigérants.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1005271 B1
- EP 0676140 A
- EP 1030558 B
- WO 2016034266 A1
- WO 2015175241 A1
- US 2014121253 A1
- US 2004198785 A1

**In der Beschreibung aufgeführte Nicht-Patentliteratur**

- **F.C. KULL et al.** *Applied Microbiology,* 1961, vol. 9, 538